# EUROPEAN PATENT APPLICATION

(11) **EP 3 588 088 A1**
(43) Date of publication of application: **01.01.2020**
(21) Application number: 18758172.3
(22) Date of filing: 12.01.2018
(51) Int. Cl.: G01N 33/533, G01N 21/64, G01N 33/48, G01N 33/53

(54) **METHOD FOR EVALUATING SURFACE STATE OF PARTICLES, AND EVALUATION SYSTEM**

(30) Priority: 27.02.2017 JP 2017034881
(71) Applicant: Konica Minolta, Inc., Tokyo 100-7015 (JP)
(72) Inventor: GOUDA, Hideki, Tokyo 100-7015 (JP); TAKANASHI, Kensaku, Tokyo 100-7015 (JP); FUTAYA, Etsuko, Tokyo 100-7015 (JP)
(74) Representative: Gille Hrabal
(86) International application number: PCT/JP2018/000660
(87) International publication number: WO 2018/154994

(57) **Abstract**

The present invention provides a method for evaluating a surface state of particles for judging suitability of colored particles used for staining a biological material. The method includes: a dispersion step of dispersing colored particles in a two-component dispersion medium containing two types of dispersion media having different polarities to form an interface; an indicator acquisition step of acquiring an indicator indicating the amount of particles contained in one of the two types of dispersion media; and a judgement step of judging, on the basis of the indicator, suitability of a surface state of the colored particles for staining a biological material. By the method for evaluating a surface state of particles according to the present invention, it is possible to evaluate a surface state of colored particles such as fluorescent nanoparticles, for example, the degree to which the surface of the colored particles has hydrophilicity with high sensitivity and stability in a short period of time without using a large amount of particles. This makes it possible to effectively judge suitability of colored particles used in staining a biological material.

## Description

### Technical Field

The present invention relates to a method and a system for evaluating a surface state of particles, and specifically to a method and a system for evaluating a surface state of particles for judging suitability of colored particles used for staining a biological material.

### Background Art

In current medical care, by analyzing the number and positions of specific proteins expressed in cancer cells, high-precision pathological diagnosis is performed at an early stage. As a method for detecting specific proteins expressed in cancer cells, a technique of bonding fluorescent nanoparticles to proteins expressed in cancer cells to label the proteins, detecting fluorescence emitted by the fluorescent nanoparticles, and thereby accurately determining the number and positions of the proteins has been developed. This technique provides a solution leading to accurate stratification of patients, improvement of a success ratio in clinical trials, and also improvement of a cure ratio of cancer and medical economic effect.

For labeling with fluorescent nanoparticles, in a case where hydrophobicity of a surface of the fluorescent nanoparticles is strong, nonspecific bonding increases to make accurate detection of a target protein difficult. Therefore, in order to prevent nonspecific bonding of fluorescent nanoparticles, the surface of the fluorescent nanoparticles is generally modified with a hydrophilic substance such as polyethylene glycol.

However, the degree of modification of the hydrophilic substance to the surface of the fluorescent nanoparticles varies depending on a treatment, and fluorescent nanoparticles with a low surface modification ratio may be produced. When labeling is performed using such fluorescent nanoparticles with a low surface modification ratio, noise increases during detection, resulting in a decrease in sensitivity.
Therefore, a method for evaluating whether or not the surface of the fluorescent nanoparticle has sufficient hydrophilicity to make it possible to detect a target protein with high accuracy is required

As such an evaluation method, for example, a method for bonding an antibody that recognizes polyethylene glycol to polyethylene glycol bonded to particles, bonding an enzyme-labeled antibody that recognizes a polyethylene glycol recognition antibody to the bonded antibody, and measuring the intensity of coloration by the enzyme with an absorptiometer, described in Patent Literature 1, is known. In addition, a method for introducing a functional group such as maleimide into a terminal of polyethylene glycol bonded to particles, developing a color using a reagent that is decomposed by a reaction with the functional machine and develops a color, and measuring the intensity of coloration with an absorptiometer, described in Patent Literature 2, is known.

However, in both the methods, for example, a measured value is unstable due to an influence of light absorption or light emission of a fluorescent dye or the like in fluorescent nanoparticles disadvantageously, a large amount of particles are required for measurement disadvantageously, and it takes a long time of about a half day to one day for measurement disadvantageously.

### Citation List

### Patent Literature

Patent Literature 1: US 2012/0015380 A1
Patent Literature 2: WO 2016/129444

### Summary of Invention

### Technical Problem

An object is to provide a method and a system for evaluating, for judging suitability of colored particles used for staining a biological material, a surface state of colored particles, for example, the degree to which the surface of the particles has a property such as hydrophilicity, the method and system obtaining a stable measured value, not requiring a large amount of particles for measurement, and not requiring a long time for measurement.

### Solution to Problem

The present invention achieving the above object provides
a method for evaluating a surface state of particles for judging suitability of colored particles used for staining a biological material, the method including:
a dispersion step of dispersing colored particles in a two-component dispersion medium containing two types of dispersion media having different polarities to form an interface and;
an indicator acquisition step of acquiring an indicator indicating the amount of particles contained in one of the two types of dispersion media; and
a judgement step of judging, on the basis of the indicator, suitability of the surface state of the colored particles for staining a biological material.

In the method for evaluating a surface state of particles, the colored particles are preferably fluorescent nanoparticles.

In the method for evaluating a surface state of particles, the indicator is preferably a fluorescence intensity.

In the method for evaluating a surface state of particles, the fluorescent nanoparticles preferably contain a fluorescent dye and particles containing a melamine resin containing the fluorescent dye.

In the method for evaluating a surface state of particles, the two types of dispersion media are preferably an aqueous dispersion medium and a dispersion medium having a polarity equal to or lower than chloroform and equal to or higher than xylene.

In the method for evaluating a surface state of particles, the two types of dispersion media are preferably an aqueous dispersion medium and chloroform, ethyl acetate, or methyl ethyl ketone.

In addition, the present invention provides
a system for evaluating a surface state of particles for judging suitability of colored particles used for staining a biological material, the system including:
a dispersion unit for dispersing colored particles in a two-component dispersion medium containing two types of dispersion media having different polarities to form an interface; and
an indicator acquisition unit for acquiring an indicator indicating the amount of particles contained in one of the two types of dispersion media.

In the system for evaluating a surface state of particles, preferably,
the colored particles are fluorescent nanoparticles,
the indicator is a fluorescence intensity, and
the indicator acquisition unit is a spectrofluorometer.

### Advantageous Effects of Invention

By the method for evaluating a surface state of particles according to the present invention, it is possible to evaluate a surface state of colored particles such as fluorescent nanoparticles, for example, the degree to which the surface of the colored particles has hydrophilicity with high sensitivity and stability in a short period of time without using a large amount of particles. This makes it possible to effectively judge suitability of colored particles used in staining a biological material.

By the system for evaluating a surface state of particles according to the present invention, the method for evaluating a surface state of particles can be efficiently performed.

### Brief Description of Drawings

Fig. 1 is a fluorescent tissue image obtained in immunostaining using fluorescent nanoparticles I having high suitability for staining a biological material.
Fig. 2 is a fluorescent tissue image obtained in immunostaining using fluorescent nanoparticles II having low suitability for staining a biological material.

### Description of Embodiments

A method for evaluating a surface state of particles according to the present invention is a method for evaluating a surface state of particles for judging suitability of colored particles used for staining a biological material, the method including:
a dispersion step of dispersing colored particles in a two-component dispersion medium containing two types of dispersion media having different polarities to form an interface;
an indicator acquisition step of acquiring an indicator indicating the amount of particles contained in one of the two types of dispersion media; and
a judgement step of judging, on the basis of the indicator, suitability of the surface state of the colored particles for staining a biological material.

The method for evaluating a surface state of particles according to the present invention uses a fact that, when particles are dispersed in a two-component dispersion medium containing two types of dispersion media having different polarities to form an interface, how the particles are dispersed in the two types of dispersion media having different polarities varies depending on surface properties thereof, measures the amount of particles dispersed in one of the two types of dispersion media, and evaluates a surface state of particles with the amount. For example, as a result of measuring the amount of particles dispersed in one of the two types of dispersion media, in a case where the amount of particles dispersed in a dispersion medium having a higher polarity is significantly larger, hydrophilicity of a particle surface is evaluated to be strong, and in a case where the amount of particles dispersed in a dispersion medium having a lower polarity is significantly larger, hydrophobicity of a particle surface is evaluated to be strong.

The method for evaluating a surface state of particles according to the present invention can be used for the purpose of evaluating what kind of surface state is possessed as a whole by an aggregate of particles in which most of the particles have an approximate surface state, and can also be used for the purpose of evaluating a ratio of particles having a certain surface state with respect to an aggregate of particles in which particles having different surface states are mixed.

Hereinafter, the method for evaluating a surface state of particles according to the present invention will be described in detail for each step.

### <Dispersion step>

In the dispersion step, colored particles are dispersed in a two-component dispersion medium containing two types of dispersion media having different polarities to form an interface.

The colored particles contain a dye such as a fluorescent dye and particles containing the dye. The dye is not particularly limited, and can be appropriately selected according to a purpose.

The particles may be organic particles or inorganic particles. Examples of the organic particles include a melamine resin, an amino resin such as a urea resin, an aniline resin, a guanamine resin, a phenol resin, a xylene resin, and a furan resin. Examples of the inorganic particles include silica particles and glass particles.

The average particle diameter of the particles is not particularly limited, but is usually 40 to 500 nm, and preferably 50 to 200 nm.

The colored particles are preferably fluorescent nanoparticles. Fluorescent nanoparticles are colored particles obtained by adding a fluorescent dye to organic particles or inorganic particles. When fluorescent nanoparticles are used as colored particles, the colored particles can be detected by fluorescence. That is, the indicator in the indicator acquisition step described later can be a fluorescence intensity.

The fluorescent dye is not particularly limited, and can be appropriately selected according to a purpose.

As the organic particles and inorganic particles in the fluorescent nanoparticles, a thermosetting resin such as a melamine resin, a urea resin, an aniline resin, a guanamine resin, a phenol resin, a xylene resin, or a furan resin is preferable. Since a thermosetting resin has a three-dimensional network structure, a dye encased therein is hardly detached from resin particles, and is suitable for labeling a protein or the like in the indicator acquisition step described later or the like. Among these resins, a melamine resin is particularly preferable because of being able to effectively suppress detachment of a dye from resin particles.

As described above, for labeling a protein, a surface of the fluorescent nanoparticles is generally modified with a hydrophilic substance such as polyethylene glycol. The method for evaluating a surface state of particles according to the present invention can be suitably used for evaluating whether or not a surface of the fluorescent nanoparticles that have been subjected to the above treatment has sufficient hydrophilicity for labeling a protein.

The dispersion medium is not particularly limited as long as being a medium for dispersing colored particles and being able to disperse the colored particles.

The two types of dispersion media having different polarities to form an interface are two types of dispersion media that are not mixed with each other due to a difference in polarity, and form an interface and are separated into two phases when being present together. The polarity can be defined by a dielectric constant.

Examples of the two types of dispersion media include an aqueous dispersion medium and an organic dispersion medium.

The aqueous dispersion medium is a dispersion medium mainly containing water, and has a water content of, for example, 90% or more, preferably 95% or more. Examples of a component other than water in the aqueous dispersion medium include an organic substance mixed with water, such as methanol or ethanol, and a salt dissolved in water. Examples of the aqueous dispersion medium include phosphate buffered saline (PBS).

The organic dispersion medium is not particularly limited as long as being able to form an interface without being mixed with the aqueous dispersion medium at normal temperature, usually at 5 to 60°C. However, when the polarity is too high or too low, a difference between dispersibility of the colored particles in the aqueous dispersion medium and dispersibility thereof in the organic dispersion medium is unlikely to appear. For example, when the polarity of the organic dispersion medium is too high, a difference in polarity from the aqueous dispersion medium is small, and both the particles having a surface with high hydrophilicity and the particles having a surface with high hydrophobicity are dispersed similarly in the aqueous dispersion medium and the organic dispersion medium. Therefore, no significant difference appears between the dispersibility of the colored particles in the aqueous dispersion medium and the dispersibility thereof in the organic dispersion medium. Meanwhile, when the polarity of the organic dispersion medium is too low, not only the particles having a surface with high hydrophilicity but also the particles having a surface with low hydrophilicity have low dispersibility in the organic dispersion medium. Therefore, no significant difference appears between the dispersibility of the colored particles in the aqueous dispersion medium and the dispersibility thereof in the organic dispersion medium.

Therefore, the aqueous dispersion medium and the organic dispersion medium have an appropriate difference in polarity from each other. The degree of a preferred difference in polarity varies depending on the property of a particle surface, the material of the particle itself, and the like, and is not uniquely determined.

In a case of evaluating whether or not a surface of the colored particles has sufficient hydrophilicity for labeling a protein by the method for evaluating a surface state of particles according to the present invention, it is preferable to use, as a dispersion medium having a smaller polarity, a dispersion medium containing 10% by weight or less of particles out of all the colored particles having a surface with sufficient hydrophilicity and containing 80% by weight or more of particles out of all the colored particles having a surface with no sufficient hydrophilicity in the dispersion medium having a smaller polarity after the dispersion step. Furthermore, it is preferable to use a dispersion medium containing 5% by weight or less of particles out of all the colored particles having a surface with sufficient hydrophilicity and containing 90% by weight or more of particles out of all the colored particles having a surface with no sufficient hydrophilicity in the dispersion medium having a smaller polarity. By using such a dispersion medium as the dispersion medium having a smaller polarity, the colored particles having a surface with sufficient hydrophilicity and the colored particles having a surface with no sufficient hydrophilicity can be clearly distinguished from each other.

In a case of evaluating whether or not a surface of the colored particles has sufficient hydrophilicity for labeling a protein by the method for evaluating a surface state of particles according to the present invention, when the colored particles are fluorescent nanoparticles containing a fluorescent dye and melamine resin particles and the two types of dispersion media are an aqueous dispersion medium and an organic dispersion medium, the organic dispersion medium is preferably a dispersion medium having a polarity equal to or lower than chloroform and a polarity equal to or higher than xylene. When the organic dispersion medium is such a dispersion medium, it can be accurately judged whether the fluorescent nanoparticles have sufficient hydrophilicity to make it possible to detect a target protein with high accuracy. That is, when the organic dispersion medium is such a dispersion medium, most of the fluorescent nanoparticles having sufficient hydrophilicity are dispersed in the aqueous dispersion medium, and most of the fluorescent nanoparticles having no sufficient hydrophilicity are dispersed in the organic dispersion medium. Therefore, particles having sufficient hydrophilicity and particles having no sufficient hydrophilicity can be clearly determined through the indicator acquisition step and the judgement step described later. Specific examples of such an organic dispersion medium include chloroform, ethyl acetate, and methyl ethyl ketone.

The two-component dispersion medium is a dispersion medium containing two types of dispersion media having different polarities to form an interface, and contains, for example, the aqueous dispersion medium and the organic dispersion medium. A ratio between the two types of dispersion media contained in the two-component dispersion medium is not particularly limited and may be appropriately determined depending on a purpose, but is usually 1 : 1.

A method for dispersing colored particles in a two-component dispersion medium is not particularly limited as long as it is possible to form a state in which particles are dispersed in the two-component dispersion medium finally. A two-component dispersion medium may be formed from the two types of dispersion media, and the colored particles may be added to and dispersed in the two-component dispersion medium. Alternatively, the colored particles may be added to one or both of the two types of dispersion media, then the two types of dispersion media may be combined to form a two-component dispersion medium, and the colored particles may be dispersed in the two-component dispersion medium. Dispersion is performed, for example, by stirring or vibrating the colored particles and the two-component dispersion medium. For dispersion, it is preferable, for example, to vigorously stir or vibrate the colored particles and the two-component dispersion medium such that the colored particles can sufficiently come into contact with each of the dispersion media. For dispersion, a tool such as a test tube, a vial, or a separatory funnel can be used appropriately.

The amounts of the two-component dispersion medium and the colored particles used in the dispersion step are not particularly limited, and it is sufficient if the amount of the two-component dispersion medium is 0.1 to 10 mL and the amount of the colored particles is 0.01 to 1.0 mg. As described above, in the method for evaluating a surface state of particles according to the present invention, it is sufficient to use a very small amount of colored particles.

### <Indicator acquisition step>

In the indicator acquisition step, an indicator indicating the amount of colored particles contained in one of the two types of dispersion media is acquired. After or without separating the two types of dispersion media with respect to one or both of the two types of dispersion media forming an interface and being in contact with each other in the two-component dispersion medium, an indicator indicating the amount of colored particles contained in the dispersion medium is acquired. For both of the two types of dispersion media, an indicator indicating the amount of colored particles contained in each of the dispersion media may be acquired.

The indicator is not particularly limited as long as being able to determine the amount of colored particles contained in the dispersion medium. For example, in a case where the colored particles are fluorescent nanoparticles, a fluorescence intensity can be used as the indicator. Here, the colored particles can also be obtained in a state of being dispersed in a water dispersion medium by appropriately manufacturing the colored particles, or can be prepared in a form of powder by drying the colored particles or as a commercial product. The amount of the colored particles, that is, the number of the colored particles can be obtained by calculating the total volume from a dry particle weight and a particle specific gravity and then dividing the total volume by a particle diameter (one particle volume). The amount of the colored particles, that is, the number of the colored particles is a value proportional to the total volume, similarly a value proportional to a dry particle weight, and similarly a value proportional to the amount of dye contained in the total particles. Therefore, the number of particles contained in one particle sample can be compared with that contained in another particle sample from the fluorescence intensity of a dye contained in each of particle samples, and a ratio of the amount of the colored particles can be determined.

Specifically, preferably, a particle sample is dispersed in a turbid solution containing two types of dispersion media of water and an organic solvent, the resulting dispersion is allowed to stand, and then a ratio between the fluorescence intensity of the water dispersion medium and the fluorescence intensity of the organic dispersion medium is determined. Alternatively, preferably, a particle sample is dispersed in an organic dispersion medium, the initial fluorescence intensity thereof is determined, then the dispersion is dispersed in a turbid solution containing two types of dispersion media according to the above, and the fluorescence intensity of the water dispersion medium is measured to determine the residual intensity of the dispersion medium of the turbid solution (a difference in intensity).

The fluorescence intensity can be obtained by performing measurement on one or both of the two type of dispersion media in a state in which the dispersion media are in contact with each other or in a state in which the dispersion media are separated from each other using a spectrofluorometer.

### <Judgement step>

The judgement step judges suitability of a surface state of the colored particles for staining of a biological material on the basis of the indicator.

The indicator obtained in the indicator acquisition step indicates the amount of particles contained in one of the two types of dispersion media. The two types of dispersion media are liquids having different polarities. In a case where the indicator indicates the amount of colored particles contained in a dispersion medium having a larger polarity, the amount of colored particles contained in the dispersion medium having a larger polarity can be determined from the indicator. If the total amount of particles is known, the amount of particles contained in a dispersion medium having a smaller polarity can also be determined. It is understood that a surface of the particles contained in the dispersion medium having a larger polarity has higher hydrophilicity than a surface of the particles contained in the dispersion medium having a smaller polarity. Therefore, from the amounts of the colored particles contained in the dispersion medium having a larger polarity and the colored particles contained in the dispersion medium having a smaller polarity, a ratio between the colored particles having a surface with higher hydrophilicity and the colored particles having a surface with lower hydrophilicity can be determined. In a case where most of the particles are particles having a surface with higher hydrophilicity, it can be evaluated that a surface of the particles as a whole has high hydrophilicity. In a case where most of the particles are particles having a surface with lower hydrophilicity, it can be judged that a surface of the particles as a whole has low hydrophilicity.

Also in a case where the indicator indicates the amount of particles contained in the dispersion medium having a smaller polarity, judgement can be made in a similar manner to the above. Also in a case where the above indicator is obtained for each of the two types of dispersion media, judgement can be made in a similar manner to the above.

The method for evaluating a surface state of particles according to the present invention only needs to perform the dispersion step, the indicator acquisition step, and the judgement step as described above, and therefore can be performed in an extremely shorter time than a conventional similar evaluation method. The method for evaluating a surface state of particles according to the present invention can be usually performed in about 0.5 hours.

The method for evaluating a surface state of particles can be performed using a system for evaluating a surface state of particles for judging suitability of colored particles used for staining a biological material, the system including: a dispersion unit for dispersing colored particles in a two-component dispersion medium containing two types of dispersion media having different polarities to form an interface; and an indicator acquisition unit for acquiring an indicator indicating the amount of particles contained in one of the two types of dispersion media. Examples of the dispersion unit include an apparatus for stirring or vibrating a test tube, a vial, and a separatory funnel. Examples of the indicator acquisition unit include a spectrofluorometer in a case where the indicator is a fluorescence intensity.

### Examples

### [Manufacturing Example]

Manufacture of particles having high suitability for staining biological material and particles having low suitability for staining biological material

### (Manufacture of fluorescent nanoparticles)

As a fluorescent dye, 14.4 mg of SulfoRhodamine 101 (manufactured by Sigma-Aldrich Co. LLC.) was added to 22 mL of water and dissolved therein. Thereafter, to this solution, 2 mL of a 5% aqueous solution of Emulgen (registered trademark) 430 (polyoxyethylene oleyl ether, manufactured by Kao Corporation) as an emulsion polymerization emulsifier was added. The temperature of this solution was raised to 70°C while the solution was stirred on a hot stirrer. Thereafter, to this solution, 0.65 g of a melamine resin raw material Nikalac MX-035 (manufactured by Nippon Carbide Industries Co., Ltd.) was added. To this solution, 1000 µL of a 10% aqueous solution of dodecylbenzenesulfonic acid (manufactured by Kanto Chemical Co., Ltd.) as a surfactant was added, and the resulting mixture was heated and stirred at 70°C for 50 minutes. Thereafter, the temperature of the resulting solution was raised to 90°C, and the solution was heated and stirred for 20 minutes. In order to remove impurities such as excess resin raw materials and a fluorescent dye from the obtained dispersion of dye resin particles, the dispersion was washed with pure water. Specifically, the dispersion was centrifuged at 20000 G for 15 minutes in a centrifuge (micro cooled centrifuge 3740 manufactured by Kubota Corporation). The supernatant was removed. Thereafter, ultrapure water was added to the resulting product, and the resulting mixture was irradiated with an ultrasonic wave to be redispersed. Washing by centrifugation, supernatant removal, and redispersion in ultrapure water was repeated five times.

By dispersing 0.1 mg of the obtained fluorescent nanoparticles in 1.5 mL of ethanol, adding 2 µL of aminopropyltrimethoxysilane (LS-3150, manufactured by Shin-Etsu Chemical Co., Ltd.) thereto, and causing a reaction for eight hours, a surface amination treatment to convert a hydroxyl group existing on a resin surface of a resin particle to an amino group was performed .

The concentration of the resulting fluorescent nanoparticles was adjusted to 3 nM using phosphate buffered saline (PBS) containing 2 mM ethylenediaminetetraacetic acid (EDTA). SM (PEG) 12 (Succinimidyl-[(N-maleomidopropionamid)-dodecaethyleneglycol] ester, manufactured by Thermo Scientific Co., Ltd.) was mixed with the dispersion of fluorescent nanoparticles having a concentration adjusted so as to have a concentration of 10 mM The resulting mixture was caused to react at 20°C for one hour to obtain a mixed solution containing maleimide-terminated fluorescent nanoparticles.

The mixture was centrifuged at 10000 G for 20 minutes, and the supernatant was removed. Thereafter, PBS containing 2 mM EDTA was added to the resulting product to disperse the precipitate, and the resulting dispersion was centrifuged again. The above washing by similar procedures was performed three times.

The manufacture of maleimide-terminated fluorescent nanoparticles as described above was performed twice to obtain fluorescent nanoparticles I and II.

### (Preparation of streptavidin)

A thiol group was added to streptavidin using streptavidin (manufactured by Wako Pure Chemical Industries, Ltd.) and N-succinimidyl S-acetylthioacetate (abbreviation: SATA), and gel filtration was performed to prepare streptavidin capable of being bonded to dye resin particles separately.

### (Bonding of resin particles to streptavidin)

The maleimide-terminated fluorescent nanoparticles I and streptavidin were mixed in PBS containing 2 mM EDTA and caused to react at room temperature for one hour, thus causing a reaction to bond the maleimide-terminated fluorescent nanoparticles I and streptavidin to each other. After the reaction, 10 mM mercaptoethanol was added thereto to stop the reaction. The resulting solution was concentrated with a centrifugal filter of ϕ0.65 µm. Thereafter, unreacted streptavidin and the like were removed using a gel filtration column for purification to obtain the fluorescent nanoparticles I bonded to streptavidin.

A similar treatment to the above was performed also for the maleimide-terminated fluorescent nanoparticle II to obtain the fluorescent nanoparticles II bonded to streptavidin.

### (Immunohistological staining)

Immunostaining of a human breast tissue was performed using a tissue staining stain containing the fluorescent nanoparticles I bonded to streptavidin and a tissue staining stain containing the fluorescent nanoparticles II bonded to streptavidin. As the tissue staining stain, a buffer such as a PBS buffer containing 1% BSA was used. As a stained section, a tissue array slide (manufactured by Cosmo Bio, product number CB-A712) was used.

After deparaffinization, the tissue array slide was subjected to displacement washing with water, and autoclaved in a 10 mM citrate buffer (pH 6.0) for 15 minutes to perform an antigen activation treatment. The tissue array slide after the antigen activation treatment was washed with a PBS buffer. Thereafter, an anti-HER2 rabbit monoclonal antibody (4B5) diluted to 0.05 nM with a PBS buffer containing 1% BSA was caused to react with the tissue section for two hours. The resulting product was washed with PBS, and then caused to react with a biotin-labeled anti-rabbit antibody diluted with PBS buffer containing 1% BSA for 30 minutes. Furthermore, the resulting product was caused to react using the above tissue staining stain, that is, was caused to react with the above-prepared dye resin particles containing streptavidin for two hours. Thereafter, the resulting product was washed to obtain immunohistochemically stained sections. The obtained immunohistochemically stained sections were immersed in a 4% neutral paraformaldehyde aqueous buffer for 10 minutes to be fixed.

### (Morphological staining)

Each of the fixed immunohistochemically stained sections was subjected to hematoxylin staining. The section after staining was immersed in ethanol to be dehydrated. The dehydrated section was further immersed in xylene to be cleared, and encapsulated with an encapsulant to be air-dried to obtain a double-stained section.

### (Evaluation of tissue image)

A fluorescent tissue image of a dye-containing resin particle was acquired using a commercially available fluorescence microscope.

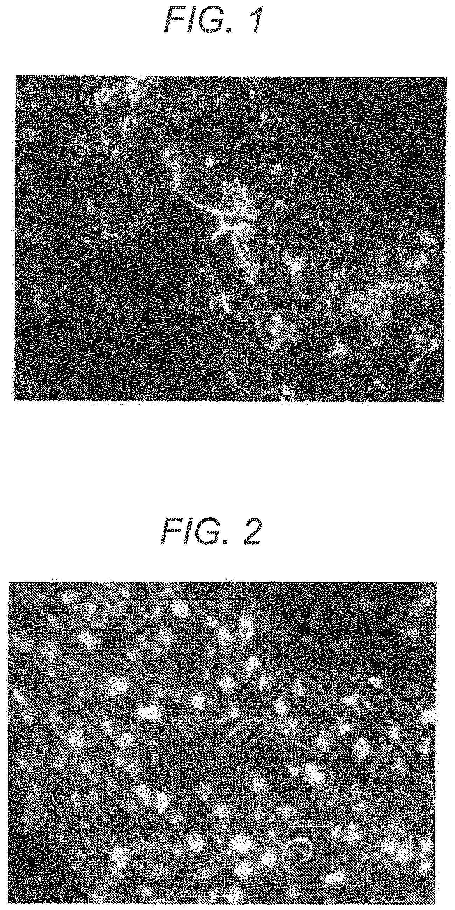

Fig. 1 illustrates a fluorescent tissue image obtained by immunostaining using a tissue staining stain containing the fluorescent nanoparticles I bonded to streptavidin. Fig. 2 illustrates a fluorescent tissue image obtained by immunostaining using a tissue staining stain containing the fluorescent nanoparticles II bonded to streptavidin.

From Fig. 1, it has been confirmed that in the immunostaining using the tissue staining stain containing the fluorescent nanoparticles I bonded to streptavidin, only a membrane of a cell is stained. From Fig. 2, it has been confirmed that in the immunostaining using the tissue staining stain containing the fluorescent nanoparticles II bonded to streptavidin, fluorescent particles are bonded to the whole cells, in particular, the fluorescent particles are bonded to nuclei.

From the above results, it has been confirmed that the fluorescent nanoparticles I have high suitability for staining a biological material, and the fluorescent nanoparticles II have low suitability for staining a biological material. It is considered that such results were obtained because the fluorescent nanoparticles I have been sufficiently treated with PEG in a manufacturing process thereof, and have a surface with high hydrophilicity, whereas the fluorescent nanoparticles II have been insufficiently treated with PEG in a manufacturing process thereof, and have a surface with low hydrophilicity.

### [Example 1]

### (Example 1-I)

In a 1.5 mL Eppendorf tube, 0.2 mL of phosphate buffered saline (PBS) was put as an aqueous dispersion medium, and 0.05 mg of the fluorescent nanoparticles I as a dry weight were dispersed therein. Furthermore, 0.2 mL of chloroform was added thereto as an organic dispersion medium. The resulting mixture was vibrated vigorously.

A PBS phase and a chloroform phase formed an interface and were separated from each other. Thereafter, the fluorescence intensity of the chloroform phase was measured at an excitation wavelength of 580 nm using a spectrofluorometer F-7000 (manufactured by Hitachi, Ltd.). From the obtained fluorescence intensity, the amount of the fluorescent nanoparticles I contained in the chloroform phase was determined using a calibration curve of the dye determined beforehand. A ratio of the amount of the fluorescent nanoparticles I contained in the chloroform phase with respect to the total amount of the fluorescent nanoparticles I was determined to be 3% by weight. That is, 97% by weight of the total fluorescent nanoparticles I was contained in the PBS phase. From this result, it has been found that the fluorescent nanoparticles I have a surface state having an extremely higher affinity to PBS than chloroform.

### (Example 1-II)

Operation similar to Example 1-I was performed except that the fluorescent nanoparticles II were used in place of the fluorescent nanoparticles I, and the amount of the fluorescent nanoparticles II contained in the chloroform phase was determined. A ratio of the amount of the fluorescent nanoparticles II contained in the chloroform phase with respect to the total amount of the fluorescent nanoparticles II was determined to be 90% by weight. That is, 10% by weight of the total fluorescent nanoparticles II was contained in the PBS phase. From this result, it has been found that the fluorescent nanoparticles II have a surface state having an extremely higher affinity to chloroform than PBS.

From the results of Examples 1-I and 1-II, it has been found that by performing the method for evaluating a surface state of particles for the fluorescent nanoparticles containing a fluorescent dye and a melamine resin using PBS and chloroform as the two types of dispersion media, it is possible to effectively distinguish between the fluorescent nanoparticles I having high suitability for staining a biological material and the fluorescent nanoparticles II having low suitability for staining a biological material.

### [Example 2]

### (Example 2-I)

Operation similar to Example 1-I was performed except that ethyl acetate was used in place of chloroform, and the amount of the fluorescent nanoparticles I contained in the ethyl acetate phase was determined. A ratio of the amount of the fluorescent nanoparticles I contained in the ethyl acetate phase with respect to the total amount of the fluorescent nanoparticles I was determined to be 6% by weight. That is, 94% by weight of the total fluorescent nanoparticles I was contained in the PBS phase. From this result, it has been found that the fluorescent nanoparticles I have a surface state having an extremely higher affinity to PBS than ethyl acetate.

### (Example 2-II)

Operation similar to Example 2-I was performed except that the fluorescent nanoparticles II were used in place of the fluorescent nanoparticles I, and the amount of the fluorescent nanoparticles II contained in the ethyl acetate phase was determined. A ratio of the amount of the fluorescent nanoparticles II contained in the ethyl acetate phase with respect to the total amount of the fluorescent nanoparticles II was determined to be 92% by weight. That is, 8% by weight of the total fluorescent nanoparticles II was contained in the PBS phase. From this result, it has been found that the fluorescent nanoparticles II have a surface state having an extremely higher affinity to ethyl acetate than PBS.

From the results of Examples 2-I and 2-II, it has been found that by performing the method for evaluating a surface state of particles for the fluorescent nanoparticles containing a fluorescent dye and a melamine resin using PBS and ethyl acetate as the two types of dispersion media, it is possible to effectively distinguish between the fluorescent nanoparticles I having high suitability for staining a biological material and the fluorescent nanoparticles II having low suitability for staining a biological material.

### [Example 3]

### (Example 3-I)

Operation similar to Example 1-I was performed except that methyl ethyl ketone was used in place of chloroform, and the amount of the fluorescent nanoparticles I contained in the methyl ethyl ketone phase was determined. A ratio of the amount of the fluorescent nanoparticles I contained in the methyl ethyl ketone phase with respect to the total amount of the fluorescent nanoparticles I was determined to be 6% by weight. That is, 94% by weight of the total fluorescent nanoparticles I was contained in the PBS phase. From this result, it has been found that the fluorescent nanoparticles I have a surface state having an extremely higher affinity to PBS than methyl ethyl ketone.

### (Example 3-II)

Operation similar to Example 3-I was performed except that the fluorescent nanoparticles II were used in place of the fluorescent nanoparticles I, and the amount of the fluorescent nanoparticles II contained in the methyl ethyl ketone phase was determined. A ratio of the amount of the fluorescent nanoparticles II contained in the methyl ethyl ketone phase with respect to the total amount of the fluorescent nanoparticles II was determined to be 85% by weight. That is, 15% by weight of the total fluorescent nanoparticles II was contained in the PBS phase. From this result, it has been found that the fluorescent nanoparticles II have a surface state having an extremely higher affinity to methyl ethyl ketone than PBS.

From the results of Examples 3-I and 3-II, it has been found that by performing the method for evaluating a surface state of particles for the fluorescent nanoparticles containing a fluorescent dye and a melamine resin using PBS and methyl ethyl ketone as the two types of dispersion media, it is possible to effectively distinguish between the fluorescent nanoparticles I having high suitability for staining a biological material and the fluorescent nanoparticles II having low suitability for staining a biological material.

### [Example 4]

### (Example 4-I)

Operation similar to Example 1-I was performed except that xylene was used in place of chloroform, and the amount of the fluorescent nanoparticles I contained in the xylene phase was determined. A ratio of the amount of the fluorescent nanoparticles I contained in the xylene phase with respect to the total amount of the fluorescent nanoparticles I was determined to be 4% by weight. That is, 96% by weight of the total fluorescent nanoparticles I was contained in the PBS phase. From this result, it has been found that the fluorescent nanoparticles I have a surface state having an extremely higher affinity to PBS than xylene.

### (Example 4-II)

Operation similar to Example 4-1 was performed except that the fluorescent nanoparticles II were used in place of the fluorescent nanoparticles I, and the amount of the fluorescent nanoparticles II contained in the xylene phase was determined. A ratio of the amount of the fluorescent nanoparticles II contained in the xylene phase with respect to the total amount of the fluorescent nanoparticles II was determined to be 3% by weight. That is, 97% by weight of the total fluorescent nanoparticles II was contained in the PBS phase. From this result, it has been found that the fluorescent nanoparticles II have a surface state having an extremely higher affinity to PBS than xylene.

From the results of Examples 4-I and 4-II, it has been found that by performing the method for evaluating a surface state of particles for the fluorescent nanoparticles containing a fluorescent dye and a melamine resin using PBS and xylene as the two types of dispersion media, it is not possible to effectively distinguish between the fluorescent nanoparticles I having high suitability for staining a biological material and the fluorescent nanoparticles II having low suitability for staining a biological material.

### [Example 5]

### (Example 5-1)

Operation similar to Example 1-1 was performed except that hexane was used in place of chloroform, and the amount of the fluorescent nanoparticles I contained in the hexane phase was determined. A ratio of the amount of the fluorescent nanoparticles I contained in the hexane phase with respect to the total amount of the fluorescent nanoparticles I was determined to be 3% by weight. That is, 97% by weight of the total fluorescent nanoparticles I was contained in the PBS phase. From this result, it has been found that the fluorescent nanoparticles I have a surface state having an extremely higher affinity to PBS than hexane.

### (Example 5-II)

Operation similar to Example 5-I was performed except that the fluorescent nanoparticles II were used in place of the fluorescent nanoparticles I, and the amount of the fluorescent nanoparticles II contained in the hexane phase was determined. A ratio of the amount of the fluorescent nanoparticles II contained in the hexane phase with respect to the total amount of the fluorescent nanoparticles II was determined to be 2% by weight. That is, 98% by weight of the total fluorescent nanoparticles II was contained in the PBS phase. From this result, it has been found that the fluorescent nanoparticles II have a surface state having an extremely higher affinity to PBS than hexane.

From the results of Examples 5-I and 5-II, it has been found that by performing the method for evaluating a surface state of particles for the fluorescent nanoparticles containing a fluorescent dye and a melamine resin using PBS and hexane as the two types of dispersion media, it is not possible to effectively distinguish between the fluorescent nanoparticles I having high suitability for staining a biological material and the fluorescent nanoparticles II having low suitability for staining a biological material.

### [Comparative Example 1]

Operation similar to Examples 1-I and 1-II was performed except that dimethyl sulfoxide was used in place of chloroform. As a result, PBS and dimethyl sulfoxide were mixed, and a phenomenon that the PBS phase and the dimethyl sulfoxide phase formed an interface and were separated from each other did not occur. For this reason, it was not possible to determine the amount of the fluorescent nanoparticles I or II contained in each of the layers.

### [Comparative Example 2]

Operation similar to Examples 1-I and 1-II was performed except that ethanol was used in place of chloroform. As a result, PBS and ethanol were mixed, and a phenomenon that the PBS phase and the ethanol phase formed an interface and were separated from each other did not occur. For this reason, it was not possible to determine the amount of the fluorescent nanoparticles I or II contained in each of the layers.

### [Comparative Example 3]

Operation similar to Examples 1-I and 1-II was performed except that acetone was used in place of chloroform. As a result, PBS and acetone were mixed, and a phenomenon that the PBS phase and the acetone phase formed an interface and were separated from each other did not occur. For this reason, it was not possible to determine the amount of the fluorescent nanoparticles I or II contained in each of the layers.

The results of the above Examples and Comparative Examples are summarized in Table 1. In Table 1, a case where evaluation of a surface state of particles was possible is indicated as "o", and a case where evaluation of a surface state of particles was not possible is indicated as "x". Furthermore, a case where the ratio of the amount of the fluorescent nanoparticles I contained in the organic dispersion medium phase with respect to the total amount of the fluorescent nanoparticles I was 10% by weight or less, and the ratio of the amount of the fluorescent nanoparticles II contained in the organic dispersion medium phase with respect to the total amount of the fluorescent nanoparticles II was 80% by weight or more was judged as "o". The other cases and a case where the aqueous dispersion medium and the organic dispersion medium were not separated from each other were judged as "x".

**[Table 1]**

| | Organic dispersion medium | Ratio of fluorescent nanoparticles I (% by mass) | Ratio of fluorescent nanoparticles II (% by mass) | Possibility of evaluation | Judgement |
|---|---|---|---|---|---|
| Example 1 | Chloroform | 3 | 90 | ○ | ○ |
| Example 2 | Ethyl acetate | 6 | 92 | ○ | ○ |
| Example 3 | Methyl ethyl ketone | 6 | 85 | ○ | ○ |
| Example 4 | Xylene | 4 | 3 | ○ | × |
| Example 5 | Hexane | 3 | 2 | ○ | × |
| Comparative Example 1 | Dimethyl sulfoxide | - | - | × | × |
| Comparative Example 2 | Hexane | - | - | × | × |
| Comparative Example 3 | Acetone | - | - | × | × |

### Industrial Applicability

By the method for evaluating a surface state of particles according to the present invention, it is possible to effectively judge whether or not a surface of colored particles such as fluorescent nanoparticles used for labeling a specific protein expressed in a cancer cell, used for pathological diagnosis, has sufficient hydrophilicity to make it possible to detect a target protein with high accuracy.

## Claims

1. A method for evaluating a surface state of particles for judging suitability of colored particles used for staining a biological material, the method comprising:
a dispersion step of dispersing colored particles in a two-component dispersion medium containing two types of dispersion media having different polarities to form an interface;
an indicator acquisition step of acquiring an indicator indicating the amount of particles contained in one of the two types of dispersion media; and
a judgement step of judging, on a basis of the indicator, suitability of a surface state of the colored particles for staining a biological material.

2. The method for evaluating a surface state of particles according to claim 1, wherein the colored particles are fluorescent nanoparticles.

3. The method for evaluating a surface state of particles according to claim 2, wherein the indicator is a fluorescence intensity.

4. The method for evaluating a surface state of particles according to claim 2 or 3, wherein the fluorescent nanoparticles contain a fluorescent dye and particles containing a melamine resin containing the fluorescent dye.

5. The method for evaluating a surface state of particles according to claim 4, wherein the two types of dispersion media are an aqueous dispersion medium and a dispersion medium having a polarity equal to or lower than chloroform and equal to or higher than xylene.

6. The method for evaluating a surface state of particles according to claim 5, wherein the two types of dispersion media are an aqueous dispersion medium and chloroform, ethyl acetate, or methyl ethyl ketone.

7. A system for evaluating a surface state of particles for judging suitability of colored particles used for staining a biological material, the system comprising:
a dispersion unit for dispersing colored particles in a two-component dispersion medium containing two types of dispersion media having different polarities to form an interface; and
an indicator acquisition unit for acquiring an indicator indicating the amount of particles contained in one of the two types of dispersion media.

8. The system for evaluating a surface state of particles according to claim 7, wherein
the colored particles are fluorescent nanoparticles,
the indicator is a fluorescence intensity, and
the indicator acquisition unit is a spectrofluorometer.
